# EUROPEAN PATENT SPECIFICATION

(11) **EP 4 192 371 B1**
(45) Date of publication and mention of the grant of the patent: **02.07.2025**
(21) Application number: 21762227.3
(22) Date of filing: 03.08.2021
(51) Int. Cl.: A61B 17/34, A61B 1/00, A61B 1/012, A61B 17/22, A61B 90/00

(54) **SYSTEMS FOR TREATING A STRICTURE ALONG THE BILIARY AND/OR PANCREATIC TRACT**
SYSTEME ZUR BEHANDLUNG EINER VERENGUNG ENTLANG DES GALLEN- UND/ODER PANKREASTRAKTES
SYSTÈMES DE TRAITEMENT D'UNE STÉNOSE LE LONG DES VOIES BILIAIRES ET/OU PANCRÉATIQUES

(30) Priority: 05.08.2020 US 202063061451 P
(43) Date of publication of application: 14.06.2023
(73) Proprietor: Boston Scientific Scimed, Inc., Maple Grove, MN 55311 (US); Northwestern University, Evanston, IL 60208 (US)
(72) Inventor: GESSLER, III, Raymond D., Roberts, Wisconsin 54023 (US); DAYTON, Peter L., Brookline, Massachusetts 02446 (US); JODA, David B., St. Paul, Minnesota 55119 (US); LEMKE, Kyle L., Hudson, Wisconsin 54016 (US); KOMANDURI, Srinadh, Downers Grove, Illinois 60515 (US); GALLUCCI, JR., Vincent, Naperville, Illinois 60565 (US); MATTESON, Jason, Beldenville, Wisconsin 54003 (US); OLSON, Mark P., New Brigton, Minnesota 55112 (US)
(74) Representative: Vossius & Partner Patentanwälte Rechtsanwälte mbB
(86) International application number: PCT/US2021/044420
(87) International publication number: WO 2022/031763

(56) References cited:
- WO-A1-97/12557
- US-A- 6 126 633
- US-A1- 2008 183 175
- US-A1- 2014 005 478
- US-A1- 2015 012 008
- US-A1- 2016 361 088
- US-A1- 2017 079 628
- US-A1- 2020 221 926

## Description

### Cross Reference to Related Applications

This application claims the benefit of and priority to U.S. Provisional Patent Application Serial No. 63/061,451 filed on August 5, 2020.

### Technical Field

The present disclosure pertains to medical devices, and example methods for manufacturing medical devices. More particularly, the present disclosure pertains to medical devices for treating strictures along the biliary and/or pancreatic tract.

### Background

A wide variety of medical devices have been developed for medical use. Some of these devices include guidewires, catheters, and the like. These devices are manufactured by any one of a variety of different manufacturing methods and may be used according to any one of a variety of methods. Of the known medical devices and methods, each has certain advantages and disadvantages. There is an ongoing need to provide alternative medical devices as well as alternative methods for manufacturing and using medical devices.

US 2015/0012008 A1 discloses a system for treating a stricture having a handle mechanism with proximal, middle and distal slidable handle members, a sheath, a steerable catheter and a needle.

### Summary

The invention is defined by claims 1 and 10. Further embodiments of the invention are defined by the dependent claims. Methods as such are not claimed.

This disclosure provides design, material, manufacturing method, and use alternatives for medical devices. A system for treating a stricture is disclosed. The system comprises: a handle having a base, a first carriage translatable relative to the base, and a second carriage translatable relative to the base; a sheath coupled to the first carriage; a catheter shaft having a proximal end region and a steerable distal end region, the proximal end region being coupled to the second carriage; a needle releasably coupled to the handle, the needle being configured to pass through tissue into a position along the biliary and/or pancreatic tract; and a steering member having a first end coupled to the steerable distal end region of the catheter shaft and a second end disposed adjacent to the handle.

Alternatively or additionally to any of the embodiments above, the handle includes a first locking member for securing the axial position of the first carriage relative to the base.

Alternatively or additionally to any of the embodiments above, the handle includes a second locking member for securing the axial position of the second carriage relative to the base, relative to the first carriage, or both.

Alternatively or additionally to any of the embodiments above, the handle includes a third locking member for securing the axial position of the steering member relative to the base, relative to the second carriage, or both.

Alternatively or additionally to any of the embodiments above, the base includes a proximal connector and wherein the needle is secured to the proximal connector.

Alternatively or additionally to any of the embodiments above, the steering member includes a ribbon wire.

Alternatively or additionally to any of the embodiments above, the catheter shaft has a lumen formed therein and wherein the steering member extends through the lumen.

Alternatively or additionally to any of the embodiments above, at least a portion of the steering member extends along an outer surface of the catheter shaft.

Alternatively or additionally to any of the embodiments above, further comprising a sleeve disposed along the portion of the steering member extending along the outer surface of the catheter shaft.

Alternatively or additionally to any of the embodiments above, further comprising an endoscope having a channel formed therein, wherein the catheter shaft is configured to extend within the channel.

A system for treating a stricture is disclosed. The system comprises: a handle having a base, a first telescoping portion coupled to the base, and a second telescoping portion coupled to the base; a sheath coupled to the first telescoping portion; a catheter shaft for non-papillary access to the biliary and/or pancreatic tract, the catheter shaft having a guidewire lumen, a proximal end region and a steerable distal end region; wherein the proximal end region is coupled to the second telescoping portion; a needle having a piercing distal end region and a proximal end region coupled to a connector disposed along the handle, the piercing distal end region needle being configured to pass through tissue into a position along the biliary and/or pancreatic tract; a steering wire having a distal end coupled to the steerable distal end region, an external portion disposed along an outer surface of the catheter shaft, and a proximal end disposed adjacent to the handle; and a sleeve disposed along the external portion of the steering wire.

Alternatively or additionally to any of the embodiments above, the handle includes a first locking member for securing the axial position of the first telescoping portion relative to the base.

Alternatively or additionally to any of the embodiments above, the handle includes a second locking member for securing the axial position of the second telescoping portion relative to the first telescoping portion.

Alternatively or additionally to any of the embodiments above, the handle includes a steering lock for securing the axial position of the steering wire relative to the base, relative to the second telescoping portion, or both.

Alternatively or additionally to any of the embodiments above, the steering wire includes a ribbon wire.

A method for crossing a stricture is disclosed. The method comprises: advancing a catheter system through a digestive tract of patient to a position adjacent to a wall surface along the digestive tract; wherein the catheter system includes catheter shaft and a needle; arranging the needle relative to the catheter shaft so that a distal end of the needle extends distally beyond a distal end of the catheter shaft; piercing through the wall surface and into a luminal position within the biliary and/or pancreatic tract adjacent to a stricture; removing the needle from the catheter system; and advancing a guidewire through the catheter shaft and past the stricture.

Alternatively or additionally to any of the embodiments above, the wall surface along the digestive tract includes a duodenum wall surface.

Alternatively or additionally to any of the embodiments above, the wall surface along the digestive tract includes a stomach wall surface.

Alternatively or additionally to any of the embodiments above, piercing through the wall surface and into a luminal position within the biliary and/or pancreatic tract adjacent to a stricture includes piercing tissue between the wall surface and the luminal position within the biliary and/or pancreatic tract adjacent.

Alternatively or additionally to any of the embodiments above, the catheter shaft includes a steering member and further comprising steering a distal end region of the catheter shaft relative to the stricture.

The above summary of some embodiments is not intended to describe each disclosed embodiment or every implementation of the present disclosure. The Figures, and Detailed Description, which follow, more particularly exemplify these embodiments.

### Brief Description of the Drawings

The disclosure may be more completely understood in consideration of the following detailed description in connection with the accompanying drawings, in which:
FIG. 1 is a side view of a system for accessing the pancreatic and/or biliary tract according to the present disclosure.
FIG. 2 is a side view of a system for accessing the pancreatic and/or biliary tract according to the present disclosure.
FIG. 3 is a cross-sectional view of a system for accessing the pancreatic and/or biliary tract according to the present disclosure.
FIG. 4 is a cross-sectional view of a catheter shaft according to the present disclosure.
FIG. 5 illustrates a catheter shaft in a curved configuration according to the present disclosure.
FIG. 6 illustrates another catheter shaft in a curved configuration according to the present disclosure.
FIG. 7 schematically depicts a portion of the digestive system.
FIGS. 8-10 illustrate a method for accessing a stricture according to the present disclosure.
FIG. 11 is a side view of a portion of an example handle according to the present disclosure.

While the disclosure is amenable to various modifications and alternative forms, specifics thereof have been shown by way of example in the drawings and will be described in detail. It should be understood, however, that the intention is not to limit the invention to the particular embodiments described. The invention is defined by the claims.

### Detailed Description

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (e.g., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the invention.

In endoscopy, a frequent medical condition arises when a patient presents with abdominal pain with or without associated jaundice. The etiology is usually some type of obstruction in the biliary tree which prevents bile from flowing naturally from the proximal tree into the duodenum. The blockage may be the result of biliary stones caught in the lumen of the ducts or a tumor which is either in the wall of the duct or impinging upon the wall from adjacent tissue. When such a stricture occurs the duct proximal to the stricture dilates and the duct distal to the stricture receives a reduced flow of bile. In order to relieve the patient's symptoms, gastroenterologists seek to find a method for resuming the flow of bile from the proximal dilated duct into the duodenum. Some interventions contemplated for reliving symptoms may include placing a stent across the stricture to drain the proximal duct, removing a stone, and/or the like.

The most common method of placing a stent across the stricture is to perform an endoscopic retrograde cholangio-pancreatography (ERCP) where a side-viewing endoscope is placed in the duodenum at the location of the biliary papilla and a guidewire is placed through the papilla and up the biliary duct, across the stricture, in a retrograde fashion. Such procedures may be challenging. For example, depending on the location, geometry, and mechanics of the stricture, deep cannulation of the proximal duct may be difficult if not be possible. Furthermore, when the physician attempts to access the biliary duct, they may inadvertently cannulate the pancreatic duct. Inadvertent cannulation of the pancreatic duct could lead to complications such as pancreatitis. Disclosed herein are devices and methods that address these and other issues, for example by utilizing antegrade (e.g., non-papillary) stricture crossing.

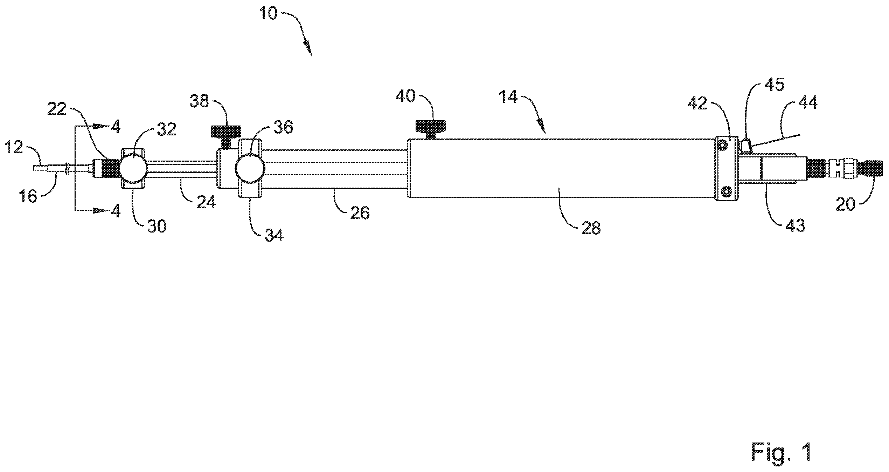

FIGS. 1-3 illustrate an example system 10 for treating a stricture along the biliary and/or pancreatic tract. In at least some instances, the system 10 is configured to be used with an endoscope (e.g., an endoscopic ultrasound device) for antegrade stricture crossing along the biliary and/or pancreatic tract. The system 10 may be understood to be a cold (e.g., unelectrified) access device that can be used for non-papillary access to the biliary and/or pancreatic tract. As will be described in more detail herein, the system 10 may be advanced through at least a portion of the digestive tract of a patient, used to pierce/puncture through a wall of the digestive tract and into a lumen/duct along the biliary and/or pancreatic tract, steered to a desired orientation, and then be used to allow a suitable device to pass therethrough for an intervention.

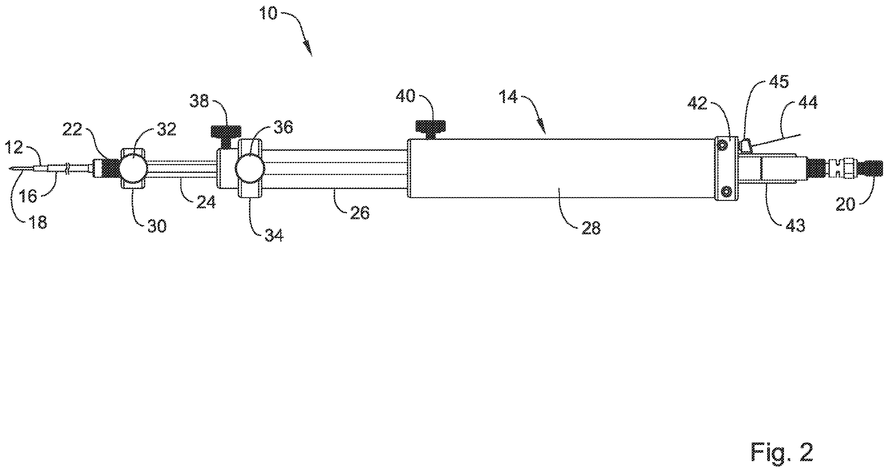

The system 10 may include a catheter shaft 12 and a handle 14 coupled to the catheter shaft 12. A sheath 16 may be coupled to the handle 14. In at least some instances, the sheath 16 may be or function like an introducer sheath. The sheath 16 may be formed form a relatively stiff material such as polyetheretherketone. Other materials are contemplated including those disclosed herein. The handle 14 may include a first or proximal connector 20. In at least some instances, the proximal connector 20 may be used to secure a device relative to the system 10. For example, a needle or sharp 18 (depicted in FIG. 2) may be releasably secured to the system 10. The needle/sharp 18 may have a piercing and/or penetrating distal end that allows the needle/sharp 18 to pass through tissue. The handle 14 may include a second or distal connector 22. In at least some instances, the distal connector 22, which may take the form of a luer or other suitable connector, may be used to secure the system 10 to an endoscope.

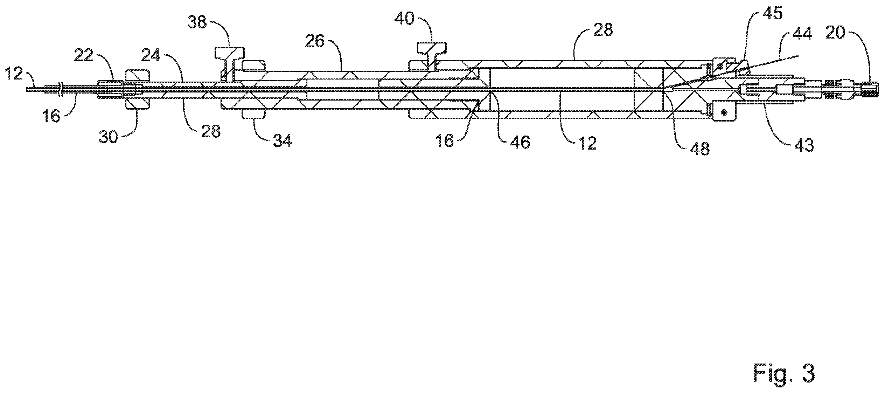

The handle 14 may include a base 24, a first telescoping member or carriage 26 translatable relative to the base 24, and a second telescoping member or carriage 28 translatable relative to the base 24 (and/or translatable relative to the first carriage 26). The base 24 and the carriages 26, 28 may be used to manipulate different portions of the system 10 during an intervention. For example, the base 24 may be coupled/secured to endoscope (e.g., the distal connector 22 may be attached to an endoscope). It may be desirable to arrange the sheath 16 relative to the endoscope. For example, it may be desirable to arrange the sheath 16 so that the sheath 16 extends just beyond the elevator of the endoscope. To adjust the sheath 16, the handle 14 may be manipulated. For example, a proximal end region 46 of the sheath 16 may be coupled to the first carriage 26 (see, for example, FIG. 3). Thus, translation of the first carriage 26 relative to the base 24 adjusts the axial position of the sheath 16 relative to the base 24 (and/or the endoscope). In some instances, the first carriage 26 may be understood to be a "length adjustment" for adjusting the length of the sheath 16 (e.g., relative to the base 24 and/or the endoscope).

A proximal end region 48 of the catheter shaft 12 may be coupled to the second carriage 28. Thus, translation of the second carriage 28 relative to the base 24 adjusts the axial position of the catheter shaft 12 relative to the base 24 (and/or the endoscope). In addition, when the needle/sharp 18 is coupled to the handle 14, the needle/sharp 18 may be secured to the proximal connector 20. In some instances, the proximal connector 20 may be coupled to the second carriage 28. Thus, when the needle/sharp 18 is secured to the handle 14, translation of the second carriage 28 relative to the base 24 adjusts the axial position of both the catheter shaft 12 and the needle/sharp 18 relative to the base 24 (and/or the endoscope). In some instances, the second carriage 28 may be understood to be a "sharp throw" for adjusting the throw or length of the needle/sharp 18 (e.g., relative to the base 24 and/or the endoscope).

The handle 14 may include a number of additional features. For example, a first stop member 30 may be disposed along the base 24. The first stop member 30 may take the form of a collar that can slide along the base 24. In at least some instances, the first stop member 30 functions as a stop or limiter that limits how far the first carriage 26 can translate along the base 24. In use, a clinician can adjust the first stop member 30 to the desired position along the base 24. The base 24 may include visual indicia or markings indicative of the relative length/position of the sheath 16 to aid the clinician in determining the suitable length/position for the first stop member 30. The first stop member 30 may include a first set screw 32. The set screw 32 may be used to secure the position of the first stop member 30 relative to the base 24.

A second stop member 34 may be disposed along the first carriage 26. The second stop member 34 may take the form of a collar that can slide along the first carriage 26. In at least some instances, the second stop member 34 functions as a stop or limiter that limits how far the second carriage 28 can translate along the first carriage 26. In use, a clinician can adjust the second stop member 34 to the desired position along the first carriage 26. The first carriage 26 may include visual indicia or markings indicative of the relative length/position of the catheter shaft 12 to aid the clinician in determining the suitable length/position for the second stop member 34. The second stop member 34 may include a second set screw 36. The second set screw 36 may be used to secure the position of the second stop member 34 relative to the first carriage 26.

The handle 14 may include a first locking member 38. The first locking member 38 may secure the axial position of the first carriage 26 relative to the base 24. In addition, the handle 14 may include a second locking member 40. The second locking member 40 may secure the axial position of the second carriage 28 relative to the first carriage 26 (and/or relative to the base 24).

The handle 14 may include a steering lock 42. The steering lock 42 may take the form of a collar that is slidable translatable along a region 43 of the handle 14. In at least some instances, the steering lock 42 may be coupled to a steering member or wire 44 coupled to the catheter shaft 12 (e.g., described in more detail herein). Translation of the steering lock 42 may actuate the steering wire 44 to cause the catheter shaft 12 to bend. When the catheter shaft 12 is in the desired shape, a set screw 45 may be actuated to secure the steering lock 42 to the handle 14 (e.g., to secure the steering lock 42 relative to the second carriage 28, the base 24, or both) and, thus secure the catheter shaft 12 in the desired shape.

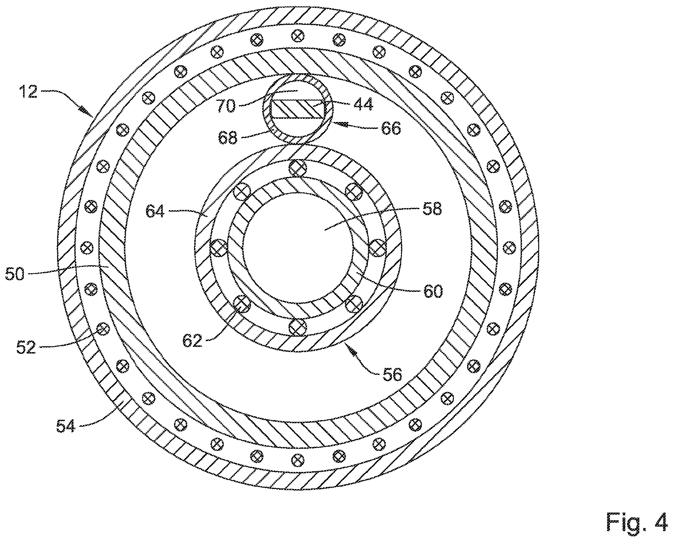

FIG. 4 is a cross-sectional view of the catheter shaft 12. Here, a number of structural features can be shown. For example, the catheter shaft 12 may include an inner layer 50, a reinforcing layer 52 (e.g., such as braid, coil, mesh, and/or the like), and an outer layer 54. The materials for the various components of the catheter shaft 12 may include those materials disclosed herein. For example, the inner layer may include a lubricious material such as polytetrafluoroethylene (PTFE), fluorinated ethylene propylene (FEP), combinations thereof, and the like, and/or other materials disclosed herein. The reinforcing layer 52 may include a stainless steel braid. The outer layer may include a polyamide, a polyether-ester, a nylon, combinations thereof, and the like, and/or other materials disclosed herein. In some instances, the materials used for the catheter shaft 12 may vary along the length of the catheter shaft 12. This may allow the catheter shaft 12 to be more flexible near the distal end thereof. In some instances, the catheter shaft 12 is formed from a single layer of material. In some instances, the catheter shaft 12 may include a tubular member with a plurality of slots formed therein. The slotted catheter shaft 12 may be formed from a nickel-titanium alloy, stainless steel, and/or the like. A sleeve of outer layer may be disposed along the slotted catheter shaft 12.

A guidewire and/or sharp tube 56, defining a lumen 58 therein, may be disposed within the catheter shaft 12. The tube 56 may include an inner layer 60, a reinforcing layer 62 (e.g., such as braid, coil, mesh, and/or the like), and an outer layer 64. The construction may of the tube 56 may be similar to the catheter shaft 12 or be different. In some instances, the tube 56 is formed from a single layer of material. A steering wire assembly 66 may also be disposed within the catheter shaft 12. The steering wire assembly 66 may include a tubular member 68 having a lumen 70 defined therein and a steering wire 44 disposed within the lumen 70. In some instances, the steering wire 44 may be a ribbon-shaped wire. In other instances, the steering wire 44 may be a round wire or have a different shape.

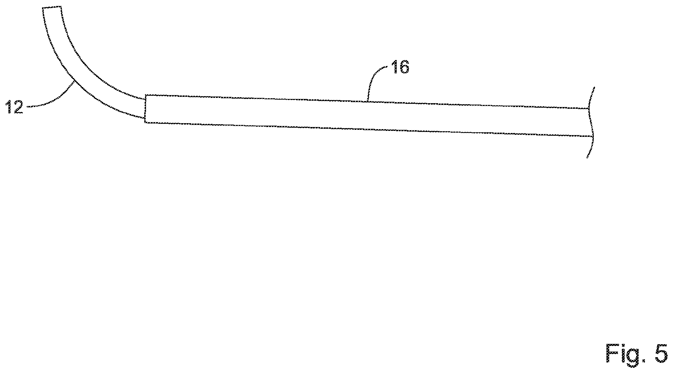

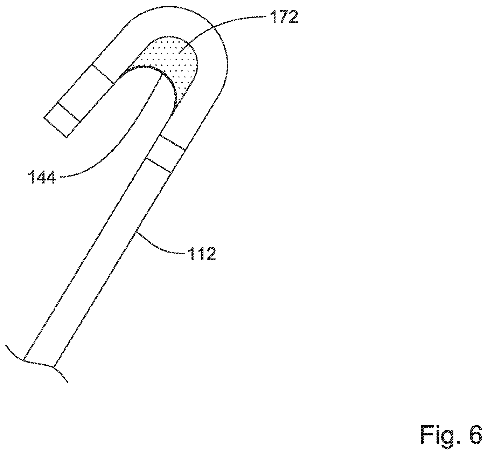

As indicated herein, the steering wire 44 may be actuated (e.g., by sliding the steering lock 42) to bend the catheter shaft 12. In some instances, the steering wire 44 may be disposed internally within the catheter shaft 12 as depicted in FIG. 5. Alternatively, a portion of the steering wire 44 may be disposed along the exterior of the catheter shaft 12. For example, FIG. 6 depicts a catheter shaft 112 with a portion of the steering wire 144 disposed along the exterior of the catheter shaft 112. In some instances, a sleeve 172 may disposed along the exterior, exposed portion of the steering wire 144, which may help to protect tissues from being damaged/cut but the steering wire 144 during use.

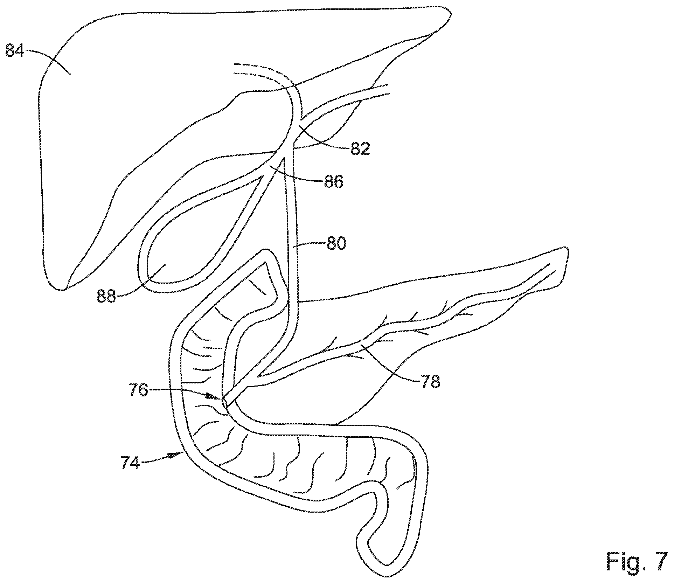

FIG. 7 illustrates an overview of the biliary system or tree. A portion of the duodenum 74 is shown. The papilla of Vater 76 (e.g., also known as the ampulla of Vater or simply the papilla) is located at the illustrated portion of the duodenum 74. The papilla 76 generally forms the opening where the pancreatic duct 78 and the common bile duct 80 can empty into the duodenum 74. The hepatic ducts, denoted by the reference numeral 82, are connected to the liver 84 and empty into the bile duct 80. Similarly, the cystic duct 86, being connected to the gall bladder 88, also empties into the bile duct 80. In general, an endoscopic or biliary procedure may include advancing a medical device to a suitable location along the biliary tree and then performing the appropriate intervention.

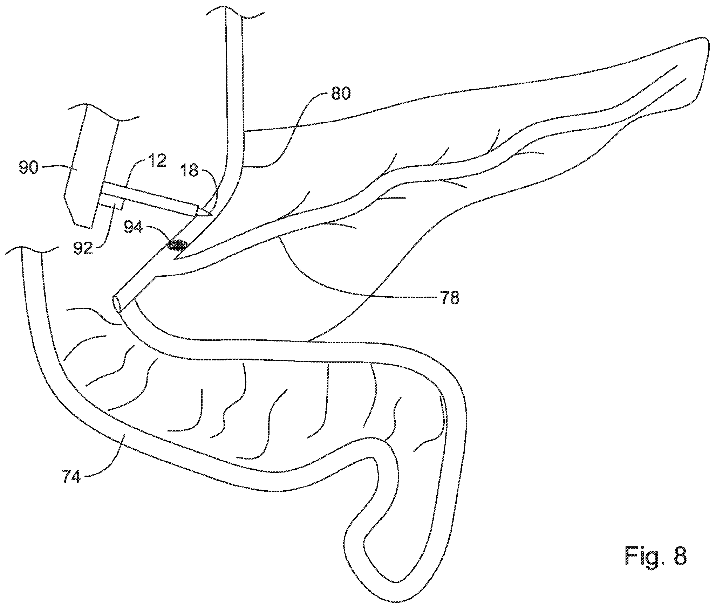

System 10 may be used to access a stricture along the pancreatic duct 78 and/or the common bile duct 80. For example, FIG. 8 illustrates an example endoscope 90 disposed in the duodenum 74. The disclosure is not intended to be limited to disposing the endoscope 90 in the duodenum 74 as other locations are contemplated including other portions of the digestive system including the stomach. The system 10 may be secured to the endoscope 90 with the catheter shaft 12 extending through a channel (e.g., a working channel) of the endoscope 90, and the handle 14 may be manipulated to position the catheter shaft 12 and sheath 16 in the desired positions. The catheter shaft 12 (e.g., having the needle/sharp 18 extending therethrough) may be advanced out from the endoscope 90. This may include the use of an elevator 92 of the endoscope 90 to direct the catheter shaft 12 toward the wall of the duodenum 74. The catheter shaft 12 having the needle/sharp 18 protruding therefrom may then pierce through the wall of the duodenum 74 and advance through tissue toward the target location, for example, a luminal position such as within common bile duct 80.

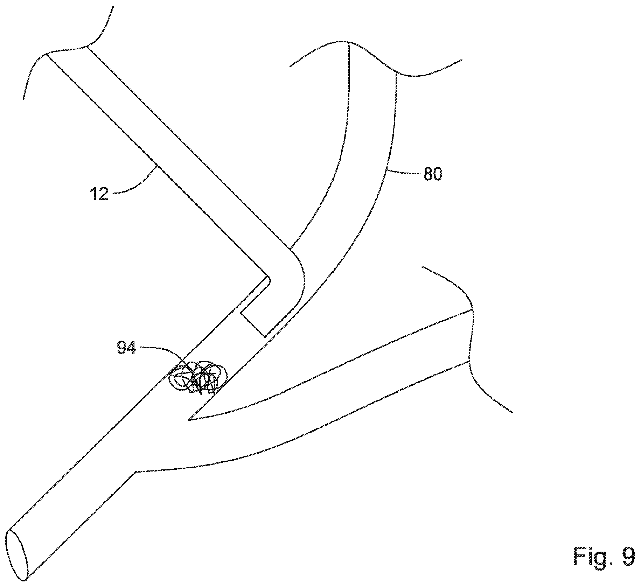

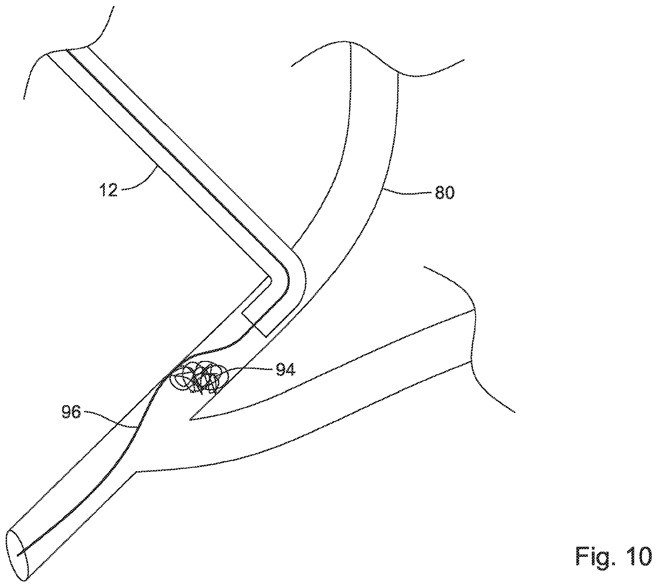

Once the catheter shaft 12 is suitably positioned, the catheter shaft 12 can be steered to the desired orientation using the steering wire 44 as schematically depicted in FIG. 9. In at least some instances, it may be desirable to remove the needle/sharp 18 from the system 10 prior to steering. Once the catheter shaft 12 is suitably oriented, a device such as a guidewire 96 may be advanced through the catheter shaft 12 and past the stricture 94 as depicted in FIG. 10.

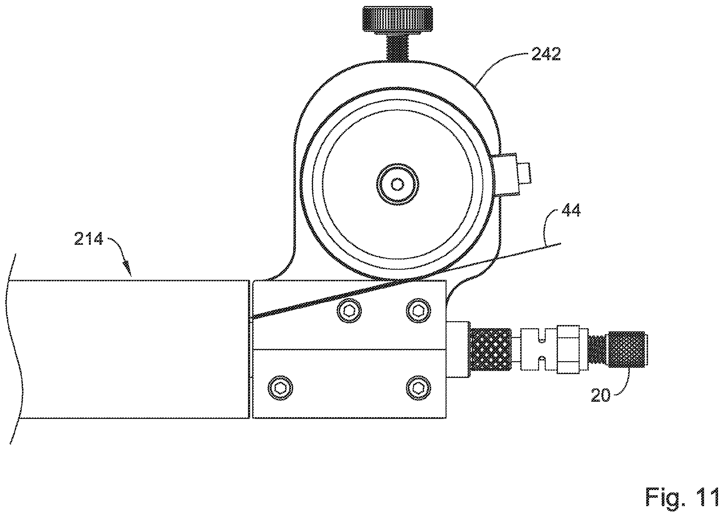

FIG. 11 is a side view of a portion of a handle 214 that can be part of a system similar to system 10. The handle 214 may be similar in form and function to handle 14. Rather than having a steering lock that slide along the handle, the handle 214 may include a rotary steering apparatus/lock 242 that is coupled to the handle 214 and to the steering wire 44. Rotation of the steering apparatus/lock 242 may actuate the steering wire 44 (e.g., and cause the catheter shaft 12 to curve/bend).

The materials that can be used for the various components of the system 10 may include those commonly associated with medical devices. For simplicity purposes, the following discussion makes reference to the catheter shaft 12. However, this is not intended to limit the devices and methods described herein, as the discussion may be applied to other similar tubular members and/or components of tubular members or devices disclosed herein.

The catheter shaft 12 may be made from or otherwise includes a metal, metal alloy, polymer (some examples of which are disclosed below), a metal-polymer composite, ceramics, combinations thereof, and the like, or other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN^{®} available from DuPont), polyether block ester, polyurethane (for example, Polyurethane 85A), polypropylene (PP), polyvinylchloride (PVC), polyether-ester (for example, ARNITEL^{®} available from DSM Engineering Plastics), ether or ester based copolymers (for example, butylene/poly(alkylene ether) phthalate and/or other polyester elastomers such as HYTREL^{®} available from DuPont), polyamide (for example, DURETHAN^{®} available from Bayer or CRISTAMID^{®} available from Elf Atochem), elastomeric polyamides, block polyamide/ethers, polyether block amide (PEBA, for example available under the trade name PEBAX^{®}), ethylene vinyl acetate copolymers (EVA), silicones, polyethylene (PE), high-density polyethylene, low-density polyethylene, linear low density polyethylene (for example REXELL^{®}), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLART), polysulfone, nylon, nylon-12 (such as GRILAMID^{®} available from EMS American Grilon), perfluoro(propyl vinyl ether) (PFA), ethylene vinyl alcohol, polyolefin, polystyrene, epoxy, polyvinylidene chloride (PVdC), poly(styrene-b-isobutylene-b-styrene) (for example, SIBS and/or SIBS 50A), polycarbonates, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL^{®} 625, UNS: N06022 such as HASTELLOY^{®} C-22^{®}, UNS: N10276 such as HASTELLOY^{®} C276^{®}, other HASTELLOY^{®} alloys, and the like), nickel-copper alloys (e.g., UNS: N04400 such as MONEL^{®} 400, NICKELVAC^{®} 400, NICORROS^{®} 400, and the like), nickel-cobalt-chromium-molybdenum alloys (e.g., UNS: R30035 such as MP35-N^{®} and the like), nickel-molybdenum alloys (e.g., UNS: N10665 such as HASTELLOY^{®} ALLOY B2^{®}), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY^{®}, PHYNOX^{®}, and the like); platinum enriched stainless steel; titanium; combinations thereof; and the like; or any other suitable material.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The invention is defined by the following claims.

## Claims

1. A system (10) for treating a stricture, the system comprising:
a handle (14; 214) having a base (24), a first carriage (26) translatable relative to the base (24), and a second carriage (28) translatable relative to the base (24);
a sheath (16) coupled to the first carriage (26);
a catheter shaft (12; 112) having a proximal end region (48) and a steerable distal end region, the proximal end region being coupled to the second carriage;
a needle (18) releasably coupled to the handle (14; 214), the needle being configured to pass through tissue into a position along the biliary and/or pancreatic tract;
a steering member (44; 144) having a first end coupled to the steerable distal end region of the catheter shaft (12; 112) and a second end disposed adjacent to the handle (14; 214);
wherein the handle (14; 214) includes a steering lock member (42, 45; 242) for securing the axial position of the steering member (44; 144) relative to the base (24), relative to the second carriage (28), or both.

2. The system (10) of claim 1, wherein the handle includes a first locking member (38) for securing the axial position of the first carriage (26) relative to the base (24).

3. The system (10) of any one of claims 1-2, wherein the handle (14; 214) includes a second locking member (40) for securing the axial position of the second carriage (28) relative to the base (24), relative to the first carriage (26), or both.

4. The system (10) of any one of claims 1-3, wherein the base (24) includes a proximal connector (20) and wherein the needle (18) is secured to the proximal connector (20).

5. The system (10) of any one of claims 1-4, wherein the steering member (44; 144) includes a ribbon wire.

6. The system (10) of any one of claims 1-5, wherein the catheter shaft (12; 112) has a lumen formed therein and wherein the steering member (44) extends through the lumen.

7. The system (10) of any one of claims 1-6, wherein at least a portion of the steering member (44) extends along an outer surface of the catheter shaft (12; 112).

8. The system (10) of claim 7, further comprising a sleeve disposed along the portion of the steering member extending along the outer surface of the catheter shaft.

9. The system (10) of any one of claims 1-8, further comprising an endoscope (90) having a channel formed therein, wherein the catheter shaft (12; 112) is configured to extend within the channel.

10. A system (10) for treating a stricture, the system (10) comprising:
a handle (14; 214) having a base (24), a first telescoping portion (26) coupled to the base (24), and a second telescoping portion (28) coupled to the base (24);
a sheath (16) coupled to the first telescoping portion (26);
a catheter shaft (12; 112) for non-papillary access to the biliary and/or pancreatic tract, the catheter shaft (12; 112) having a guidewire lumen, a proximal end region (48) and a steerable distal end region;
wherein the proximal end region (48) is coupled to the second telescoping portion (28);
a needle (18) having a piercing distal end region and a proximal end region coupled to a connector (20) disposed along the handle (14; 214), the piercing distal end region needle (18) being configured to pass through tissue into a position along the biliary and/or pancreatic tract;
a steering wire (44; 144) having a distal end coupled to the steerable distal end region, an external portion disposed along an outer surface of the catheter shaft, and a proximal end disposed adjacent to the handle (14; 214); and
a sleeve (172) disposed along the external portion of the steering wire (44; 144);
wherein the handle (14; 214) includes a steering lock (42; 242) for securing the axial position of the steering wire (44; 144) relative to the base (24), relative to the second telescoping portion (28), or both.

11. The system (10) of claim 11, wherein the handle (14; 214) includes a first locking member (38) for securing the axial position of the first telescoping portion (26) relative to the base (24).

12. The system (10) of any one of claims 11-12, wherein the handle (14; 214) includes a second locking member (40) for securing the axial position of the second telescoping portion (28) relative to the first telescoping portion (26).

13. The system (10) of any one of claims 11-12, wherein the steering wire (44; 144) includes a ribbon wire.

## Patentansprüche

1. System (10) zur Behandlung einer Verengung, wobei das System aufweist:
einen Griff (14; 214) mit einer Basis (24), einem relativ zu der Basis (24) verschiebbaren ersten Schlitten (26) und einem relativ zu der Basis (24) verschiebbaren zweiten Schlitten (28);
eine mit dem ersten Schlitten (26) gekoppelte Hülle (16);
einen Katheterschaft (12; 112) mit einem proximalen Endbereich (48) und einem steuerbaren distalen Endbereich, wobei der proximale Endbereich mit dem zweiten Schlitten gekoppelt ist;
eine auf lösbare Weise mit dem Griff (14; 214) gekoppelte Nadel (18), wobei die Nadel konfiguriert ist, um Gewebe zu durchdringen und in eine Position entlang des Gallen- und/oder Pankreastrakts zu gelangen;
ein Steuerelement (44; 144), das ein mit dem steuerbaren distalen Endbereich des Katheterschafts (12; 112) gekoppeltes erstes Ende und ein angrenzend an den Griff (14; 214) angeordnetes zweites Ende hat;
wobei der Griff (14; 214) aufweist: ein Steuerverriegelungselement (42, 45; 242) zum Fixieren der axialen Position des Steuerelements (44; 144) relativ zu der Basis (24) und/oder relativ zu dem zweiten Schlitten (28).

2. System (10) nach Anspruch 1, wobei der Griff aufweist: ein erstes Verriegelungselement (38) zum Fixieren der axialen Position des ersten Schlittens (26) relativ zu der Basis (24).

3. System (10) nach einem der Ansprüche 1 bis 2, wobei der Griff (14; 214) aufweist: ein zweites Verriegelungselement (40) zum Fixieren der axialen Position des zweiten Schlittens (28) relativ zu der Basis (24) und/oder relativ zu dem ersten Schlitten (26).

4. System (10) nach einem der Ansprüche 1 bis 3, wobei die Basis (24) einen proximalen Verbinder (20) aufweist, und wobei die Nadel (18) an dem proximalen Verbinder (20) befestigt ist.

5. System (10) nach einem der Ansprüche 1 bis 4, wobei das Steuerelement (44; 144) einen Banddraht aufweist.

6. System (10) nach einem der Ansprüche 1 bis 5, wobei in dem Katheterschaft (12; 112) ein Lumen gebildet ist, und wobei das Steuerelement (44) sich durch das Lumen erstreckt.

7. System (10) nach einem der Ansprüche 1 bis 6, wobei mindestens ein Teil des Steuerelements (44) sich entlang einer Außenfläche des Katheterschafts (12; 112) erstreckt.

8. System (10) nach Anspruch 7, ferner aufweisend eine Manschette, die entlang des sich entlang der Außenfläche des Katheterschafts erstreckenden Teils des Steuerelements angeordnet ist.

9. System (10) nach einem der Ansprüche 1 bis 8, ferner aufweisend ein Endoskop (90) mit einem darin gebildeten Kanal, wobei der Katheterschaft (12; 112) konfiguriert ist, um sich in dem Kanal zu erstrecken.

10. System (10) zur Behandlung einer Verengung, wobei das System (10) aufweist:
einen Griff (14; 214) mit einer Basis (24), einem mit der Basis (24) gekoppelten ersten teleskopischen Teil (26) und einem mit der Basis (24) gekoppelten zweiten teleskopischen Teil (28);
eine mit dem ersten teleskopischen Teil (26) gekoppelte Hülle (16);
einen Katheterschaft (12; 112) für einen nichtpapillaren Zugang zu dem Gallen- und/oder Pankreastrakt, wobei der Katheterschaft (12; 112) ein Führungsdrahtlumen, einen proximalen Endbereich (48) und einen steuerbaren distalen Endbereich hat;
wobei der proximale Endbereich (48) mit dem zweiten teleskopischen Teil (28) gekoppelt ist;
eine Nadel (18) mit einem stechenden distalen Endbereich und einem proximalen Endbereich, der mit einem Verbinder (20) gekoppelt ist, der entlang des Griffs (14; 214) angeordnet ist, wobei der stechende distale Endbereich der Nadel (18) konfiguriert ist, um Gewebe zu durchdringen und in eine Position entlang des Gallen- und/oder Pankreastrakts zu gelangen;
einen Steuerdraht (44; 144), der ein mit dem steuerbaren distalen Endbereich gekoppeltes distales Ende, einen entlang einer Außenfläche des Katheterschafts angeordneten externen Teil und ein angrenzend an den Griff (14; 214) angeordnetes proximales Ende hat; und
eine Manschette (172), die entlang des externen Teils des Steuerdrahts (44; 144) angeordnet ist;
wobei der Griff (14; 214) aufweist: eine Steuerverriegelung (42; 242) zum Fixieren der axialen Position des Steuerdrahts (44; 144) relativ zu der Basis (24) und/oder relativ zu dem zweiten teleskopischen Teil (28).

11. System (10) nach Anspruch 10, wobei der Griff (14; 214) aufweist: ein erstes Verriegelungselement (38) zum Fixieren der axialen Position des ersten teleskopischen Teils (26) relativ zu der Basis (24).

12. System (10) nach einem der Ansprüche 11 bis 12, wobei der Griff (14; 214) aufweist: ein zweites Verriegelungselement (40) zum Fixieren der axialen Position des zweiten teleskopischen Teils (28) relativ zu dem ersten teleskopischen Teil (26).

13. System (10) nach einem der Ansprüche 11 bis 12, wobei der Steuerdraht (44; 144) einen Banddraht aufweist.

## Revendications

1. Système (10) pour traiter une sténose, le système comprenant :
une poignée (14 ; 214) ayant une base (24), un premier chariot (26) pouvant effectuer une translation par rapport à la base (24) et un deuxième chariot (28) pouvant effectuer une translation par rapport à la base (24) ;
une gaine (16) couplée au premier chariot (26) ;
une tige de cathéter (12 ; 112) ayant une région d'extrémité proximale (48) et une région d'extrémité distale dirigeable, la région d'extrémité proximale étant couplée au deuxième chariot ;
une aiguille (18) couplée, de manière amovible, à la poignée (14 ; 214), l'aiguille étant configurée pour passer à travers le tissu dans une position le long des voies biliaires et/ou pancréatiques ;
un élément de direction (44 ; 144) ayant une première extrémité couplée à la région d'extrémité distale dirigeable de la tige de cathéter (12 ; 112) et une deuxième extrémité disposée de manière adjacente à la poignée (14 ; 214) ;
dans lequel la poignée (14 ; 214) comprend un élément de verrouillage de direction (42, 45 ; 242) pour fixer la position axiale de l'élément de direction (44 ; 144) par rapport à la base (24), par rapport au deuxième chariot (28) ou aux deux.

2. Système (10) selon la revendication 1, dans lequel la poignée comprend un premier élément de verrouillage (38) pour fixer la position axiale du premier chariot (26) par rapport à la base (24).

3. Système (10) selon l'une quelconque des revendications 1 à 2, dans lequel la poignée (14 ; 214) comprend un deuxième élément de verrouillage (40) pour fixer la position axiale du deuxième chariot (28) par rapport à la base (24), par rapport au premier chariot (26) ou aux deux.

4. Système (10) selon l'une quelconque des revendications 1 à 3, dans lequel la base (24) comprend un connecteur proximal (20) et dans lequel l'aiguille (18) est fixée au connecteur proximal (20).

5. Système (10) selon l'une quelconque des revendications 1 à 4, dans lequel l'élément de direction (44 ; 144) comprend un fil plat.

6. Système (10) selon l'une quelconque des revendications 1 à 5, dans lequel la tige de cathéter (12 ; 112) a une lumière formée dans cette dernière et dans lequel l'élément de direction (44) s'étend à travers la lumière.

7. Système (10) selon l'une quelconque des revendications 1 à 6, dans lequel au moins une partie de l'élément de direction (44) s'étend le long d'une surface externe de la tige de cathéter (12 ; 112).

8. Système (10) selon la revendication 7, comprenant en outre un manchon disposé le long de la partie de l'élément de direction s'étendant le long de la surface externe de la tige de cathéter.

9. Système (10) selon l'une quelconque des revendications 1 à 8, comprenant en outre un endoscope (90) ayant un canal formé dans ce dernier, dans lequel la tige de cathéter (12 ; 112) est configurée pour s'étendre à l'intérieur du canal.

10. Système (10) pour traiter une sténose, le système (10) comprenant :
une poignée (14 ; 214) ayant une base (24), une première partie télescopique (26) couplée à la base (24) et une deuxième partie télescopique (28) couplée à la base (24) ;
une gaine (16) couplée à la première partie télescopique (26) ;
une tige de cathéter (12 ; 112) pour l'accès non papillaire aux voies biliaires et/ou pancréatiques, la tige de cathéter (12 ; 112) ayant une lumière de fil-guide, une région d'extrémité proximale (48) et une région d'extrémité distale dirigeable ;
dans lequel la région d'extrémité proximale (48) est couplée à la deuxième partie télescopique (28) ;
une aiguille (18) ayant une région d'extrémité distale de perforation et une région d'extrémité proximale couplée à un connecteur (20) disposé le long de la poignée (14 ; 214), l'aiguille à région d'extrémité distale de perforation (18) étant configurée pour traverser le tissu dans une position le long des voies biliaires et/ou pancréatiques ;
un fil de direction (44 ; 144) ayant une extrémité distale couplée à la région d'extrémité distale dirigeable, une partie externe étant disposée le long d'une surface externe de la tige de cathéter, et une extrémité proximale disposée de manière adjacente à la poignée (14 ; 214) ; et
un manchon (172) disposé le long de la partie externe du fil de direction (44 ; 144) ;
dans lequel la poignée (14 ; 214) comprend un verrouillage de direction (42 ; 242) pour fixer la position axiale du fil de direction (44 ; 144) par rapport à la base (24), par rapport à la deuxième partie télescopique (28) ou aux deux.

11. Système (10) selon la revendication 10, dans lequel la poignée (14 ; 214) comprend un premier élément de verrouillage (38) pour fixer la position axiale de la première partie télescopique (26) par rapport à la base (24).

12. Système (10) selon l'une quelconque des revendications 11 à 12, dans lequel la poignée (14 ; 214) comprend un deuxième élément de verrouillage (40) pour fixer la position axiale de la deuxième partie télescopique (28) par rapport à la première partie télescopique (26).

13. Système (10) selon l'une quelconque des revendications 11 à 12, dans lequel le fil de direction (44 ; 144) comprend un fil plat.
